# EUROPEAN PATENT SPECIFICATION

(11) **EP 1 508 335 B1**
(45) Date of publication and mention of the grant of the patent: **28.09.2011**
(21) Application number: 03730647.9
(22) Date of filing: 27.05.2003
(51) Int. Cl.: A61K 36/185, A61K 31/20, A61K 31/201, A61P 1/02, A61P 31/04

(54) **COMPOSITIONS COMPRISING A COCOA FRACTION FOR USE IN THE TREATMENT OF PERIODONTITIS CAUSED BY BACTERIA BELONGING TO GENUS PORPHYROMONAS, PREVOTELLA, FUSOBACTERIUM OR ACTINOBACILLUS**
ZUSAMMENSETZUNGEN ENTHALTEND EINE KAKAOFRAKTION ZUR BEHANDLUNG VON PERIODONTITIS VERURSACHT VON BAKTERIEN DER GATTUNGEN PORPHYROMONAS, PREVOTELLA, FUSOBACTERIUM ODER ACTINOBACILLUS
COMPOSITION COMPRENANT UNE FRACTION DE CACAO POUR LE TRAITEMENT DU PÉRIODONTE CAUSÉ PAR DES BACTÉRIES DU GENRE PORPHYROMONAS, PREVOTELLA, FUSOBACTERIUM OU ACTINOBACILLUS

(30) Priority: 27.05.2002 JP 2002152185
(43) Date of publication of application: 23.02.2005
(73) Proprietor: MORINAGA & CO. LTD., Tokyo 108-0014 (JP)
(72) Inventor: NISHIMURA, Eisaku, Res. Inst. Morinaga & Co., Ltd., Yokohama-shi, Kanagawa 230-8504 (JP); HIRAO, Chinami, Res. Inst. Morinaga & Co., Ltd., Yokohama-shi, Kanagawa 230-8504 (JP); OHSHIMA, Tomoko, Dept. of Oral Bact. Tsurumi Uni., Yokohama-shi, Kanagawa 230-8501 (JP); SATO, Susumu, Res. Inst. Morinaga & Co., Ltd., Yokohama-shi, Kanagawa 230-8504 (JP); INAGAKI, Hiroyuki, Res. Inst. Morina& Co., Ltd., Yokohama-shi, Kanagawa 230-8504 (JP); KAMEI, Masanori, Res. Inst. Morinaga & Co., Ltd., yokohama-shi, Kanagawa 230-8504 (JP); KATO, Masatoshi, Res. Inst. Morinaga& Co., Ltd., Yokohama-shi, Kanagawa 230-8504 (JP); HASHIZUME, Shuichi, Morinaga Inst. of Biol.Sci., Yokohama-shi, Kanagawa 230-8504 (JP); MAEDA, Nobuko, Dept. of Oral Bact. Tsurumi Uni., Yokohama-shi, Kanagawa 230-8501 (JP)
(74) Representative: Beacham, Annabel Rose
(86) International application number: PCT/JP2003/006614
(87) International publication number: WO 2003/099304

(56) References cited:
- WO-A1-98/09533
- JP-A- 7 238 028
- JP-A- 2000 128 801
- JP-A- 2000 128 801
- JP-A- 2003 089 641
- OSAWA K ET AL: "Identification of cariostatic substances in the cacao bean husk: Their anti-glucosyltransferase and antibacterial activities." JOURNAL OF DENTAL RESEARCH, vol. 80, no. 11, November 2001 (2001-11), pages 2000-2004, XP002455753 ISSN: 0022-0345
- OOSHIMA T ET AL: "Caries inhibitory activity of cacao bean husk extract in in-vitro and animal experiments" ARCHIVES OF ORAL BIOLOGY, vol. 45, no. 8, August 2000 (2000-08), pages 639-645, XP002455754 ISSN: 0003-9969
- MASANORI KAMEI ET AL.: 'Antibacterial effects of cocoa on helicobacter pylori and results of clinical trials (cocoa no helicobacter pylori sakkin koka oyobi rinsho shiken)' BIOINDUSTRY vol. 18, no. 12, 2001, pages 5 - 14, XP002971805
- NOBUYUKI TANI ET AL.: 'Studies on antibacterial effect of root canal sealer (NU-59) (nekan jutenyo sealer NU-59 no kikin koka)' NIHON SHIKA HOZONGAKU ZASSHI vol. 31, no. 5, 1988, pages 1481 - 1488, XP002971806
- FUMINORI KANETSUNA ET AL.: 'Bactericidal effect of fatty acids on mycobactera, with particular reference to the suggested mechanism of intracellular killing' MICROBIOL. IMMUNOL. vol. 29, no. 2, 1985, pages 127 - 141, XP002971807
- SOUICHI OHTA ET AL.: 'Antibiotic activity of unsaturated fatty acids on methicillin-resistant staphylococcus aureus' BIOSCI. BIOTECH. BIOCHEM. vol. 57, no. 12, 1993, pages 2194 - 2195, XP001149161
- MUELLER KARL-DIETER ET AL.: 'Characterization of the periodontal microflora by the fatty acid profile of the broth-grown microbial population' ZENTRALBLATT FUER BAKTERIOLOGIE vol. 288, no. 4, 1998, pages 441 - 449, XP002971808

## Description

### Technical Field

The present invention relates to a composition with antibacterial activity for use against periodontal disease bacteria, containing a cocoa fraction derived from cacao mass as an active ingredient, and a food or drink for antibacterial activity for use against periodontal disease bacteria and a composition for cleaning oral cavity, containing the composition.

### Background Art

Periodontal diseases such as periodontitis and periodontoclasia are main causes of loss of teeth for adult persons. Although the Ministry of Health, Labor and Welfare of Japan has also been involved in the prevention of periodontal diseases since 1988, the morbidity of the disease has been increased year by year. For example, about 80% of Japanese of from 35 to 64 years old are suspected to have gingivitis (primary stage of periodontal disease) or periodontitis (late stage of periodontal disease), and it is reported that the morbidity of gingivitis even among young generations of from 25 to 34 years old reaches up about 60% and that of periodontitis reaches at least 10%.

According to the researches of American Academy of Periodontology, it is found that periodontal disease patients have a risk of suffering myocardinal infarction about 2.8 times as high as healthy persons, and that, in case of women, the risk of giving premature birth is up about 7.5 times higher. Furthermore, it has been said that such persons are susceptible to respiratory diseases such as pneumonia, asthma and pharyngitis, or having difficulties in controlling the blood glucose level, which often become the cause of worsening diabetes.

Main cause of periodontal disease is bacteria in dental plague deposited in periodontal pocket. In the periodontal pocket of normal healthy persons, Gram-positive bacteria usually account for the most of the bacteria present therein. However, as the periodontal disease progresses, the cells of Gram-negative bacteria, such as *Porphyromonas (Bacteroides) gingivalis, Fusobacterium nucleatum,* and *Actinobacillus actinomycetemcomitans,* tend to increase in their number. Gram-negative bacteria, particularly *Porphyromonas gingivalis* is detected at a high rate among severe adult periodontal disease patients from the focus, and in many cases, the antibody titer in such patients' blood serum against the bacteria tend to increase. Further it has been shown that an inoculation of *Porphyromonas gingivalis* cells to animals leads to the aggravation of periodontal inflammation. From these results, it is suggested that *Porphyromonas gingivalis* plays an important role in the beginning and progress of periodontal disease, and thus it is thought to be the most likely pathogen causing periodontal disease (periodontal disease bacteria).

As for the method for relief from symptoms accompanying periodontal disease, anti-inflammatory effects of some medicaments for prevention or therapy of periodontal inflammation may be mentioned. However, such method is not more than a symptomatic treatment. On the contrary, the prevention or treatment by eradicating of bacteria cells is more fundamental method in order to eliminate the cause.

Accordingly, for getting relief from symptoms accompanying periodontal disease, it is important to inhibit the growth of Gram-negative bacteria such as *Porphyromonas gingivalis* in the periodontal pocket. Therefore, dentifrices and mouthwashes having various kinds of antibacterial substances effective against such bacteria incorporated, have been developed and commercially available.

For example, JP-A-3-218320 (Patent Document 1) discloses a preventive for periodontal disease and mouth odor, which contains extracts obtained from tea by extraction with water or hot water, or extracts obtained from tea by extraction with a hydrophilic organic solvent such as ethanol, as active ingredient.

Further, JP-A-4-77424 (Patent Document 2) discloses a medicament for antibacterial activity against periodontal disease bacteria, which has a growth inhibitory activity specific to the periodontal disease pathogenic bacteria *Bacteroides gingivalis*, and also exhibits an inhibitory effect against collagenase activity derived from *Bacteroides gingivalis*. It contains catechins extracted from tea (Cameria sinensis) such as gallic acid, (+)-catechin, (+)-gallocatechin,
(-)-epicatechin, (-)-epigallocatechin,
(-)-epicatechingallate or (-)-epigallocatechingallate, or dimers, trimers or the like thereof, as an active ingredient.

Further, JP-A-6-56687 (Patent Document 3) discloses a medicament for removing dental plaque and for preventing a calculus deposition, which contains extracts obtained from tealeaves by extraction with water or hot water, or extracts obtained from tealeaves by extraction with a hydrophilic organic solvent such as ethanol, as active ingredient.

Further, JP-A-7-238028 (Patent Document 4) discloses a medicament for preventing a dental plaque deposition, which contains extracts and/or their decomposed derivatives obtained from alkaline-chemical treated cacao mass by extraction with a polar solvent, or extracts and/or their decomposed derivatives obtained from cocoa powder that is a resultant after removing fat or oil from the cacao mass by extraction with a polar solvent.

Further, JP-A-8-81380 (Patent Document 5) discloses a medicament for antibacterial activity against periodontal disease bacteria, which contains polyphenols exhibiting a weight average molecular weight of 800 to 10,000 and a degree of polymerization of 3 to 30, as an active ingredient.

Although it was often proposed that the extracts from tea could use as an active ingredient for a medicament for antibacterial activity against periodontal disease bacteria, as disclosed in the above Patent Documents 1 to 3 and 5, it has never been reported that a cocoa fraction contained in cacao mass and its extracts have an antibacterial effect against the periodontal disease bacteria. For example, the above Patent Document 4 discloses that extracts and/or their decomposed derivatives obtained from alkaline-chemical treated cacao mass by extraction with a polar solvent, and extracts and/or their decomposed derivatives obtained from cocoa powder that is a resultant after removing fat or oil from the cacao mass by extraction with a polar solvent, are capable of suppressing the adhesion of glucan onto teeth (development of dental plaque) caused by *Streptococcus mutans* that is known as the pathogenic bacteria of dental cavity. However, Patent Document 4 discloses nothing about the antibacterial activity against the periodontal disease bacteria.

Besides, the effectiveness of antibacterial substances conventionally incorporated in a dentifrice, a mouthwash, etc. has not been sufficient for bactericidal effects in many cases. On the other hand, for example, antibiotics such as tetracycline have excellent antibacterial activities, but the use thereof is restricted due to the outbreak of resistant bacteria or the problem of side effects, etc. Further, the antibiotics inhibit the growth of not only the periodontal disease bacteria but also nonpathogenic bacteria indigenous in the oral cavity, thereby adversely affecting the balance of indigenous bacteria flora in the oral cavity.

As mentioned above, therapy of periodontal disease is one of the most difficult problems to solve in dentistry, and no effective prevention of periodontal disease has been found so far. Therefore prevention of periodontal disease will become an important issue since aging society is advancing in the future.

Accordingly, it is an object of the present invention to provide a composition for antibacterial activity against periodontal disease bacteria, a food or drink for antibacterial activity against periodontal disease bacteria, and a composition for cleaning oral cavity, which have a high safety without showing any side effects, and which exhibit excellent bactericidal effects against the periodontal disease bacteria without influencing the growth of nonpathogenic bacteria indigenous in the oral cavity.

### Disclosure of the Invention

The present inventors have made intensive studies to accomplish the above object, and as a result, found that cocoa inhibits the growth of the periodontal disease bacteria such as *Porphyromonas gingivalis* and has excellent antibacterial activity against periodontal disease bacteria. The present invention has been accomplished on the basis of this discovery.

The first aspect of the present invention is to provide a composition comprising a cocoa fraction as an active ingredient derived from cacao mass for use in treating or preventing periodontal disease caused by periodontal disease bacteria belonging to *genus Porphyromonas, genus Prevotella, genus Fusobacterium* or *genus Actinobacillus.*

According to the first aspect, a composition for antibacterial activity against periodontal disease bacteria, which is safe and exhibits excellent bactericidal effects without any concerns about side effects, can be provided because the active ingredient is the cocoa fraction that has been taken as the starting material of chocolate or cocoa from the old days. Further, the cocoa fraction is an excellent material from the aspect of nutrition, and also has bactericidal effects against *H. pyrori* (see Japanese Patent No. 3110020), whereby alimentation effects and health maintenance effects can also be expected at the same time.

The second aspect of the present invention is to provide a composition of the invention which comprises a hot water-extract of a cocoa fraction as an active ingredient derived from cocao mass.

According to the second aspect, it is possible to provide a composition for antibacterial activity against periodontal disease bacteria exhibiting further excellent bactericidal effects. Further, since the hot water-extract of the cocoa fraction does not contain insoluble components, it can easily be incorporated into a drink, food, etc.

The third aspect of the present invention is to provide a composition of the invention which comprises polyphenols extracted from a cocoa fraction as an active ingredient derived from cocao mass.

The fourth aspect of the present invention is to provide a composition of the invention which comprises free fatty acids extracted from a cocoa fraction as an active ingredient derived from cacoa mass.

The fifth aspect of the present invention is to provide a composition of the invention which comprises polyphenols and free fatty acids extracted from a cocoa fraction as active ingredients derived from cacao mass.

According to the third, fourth and fifth aspects, it is possible to provide compositions for antibacterial activity against periodontal disease bacteria exhibiting excellent bactericidal effects with easiness in handling.

The sixth aspect of the present invention is to provide the composition for antibacterial activity against periodontal disease bacteria according to the aspects 4 or 5, wherein the free fatty acid is at least one selected from palmitic acid, stearic acid, oleic acid and linoleic acid.

The seventh aspect of the present invention is to provide the composition for antibacterial activity against periodontal disease bacteria according to any one of the aspects 1 to 6, wherein the cocoa fraction is cocoa powder and/or cacao mass.

The eighth aspect of the present invention is to provide the composition for antibacterial activity against periodontal disease bacteria according to any one of the aspects 1 to 7, wherein the composition exhibits antibacterial effects against periodontal disease bacteria without inhibiting growth of nonpathogenic bacteria indigenous in oral cavity.

The compositions of the invention are for use in treating or preventing periodontal disease caused by periodontal disease bacteria belonging to *genus Porphyromonas, genus Prevotella, genus Fusobacterium* or *genus Actinobacillus*.

The composition for antibacterial activity against periodontal disease bacteria in the present invention enables to efficiently eradicate the cells of the periodontal disease bacteria those are often detected from the focus of periodontal disease at a high rate in patients without inhibiting the growth of nonpathogenic bacteria indigenous in the oral cavity.

The tenth aspect of the present invention is to provide a composition for use as defined in any one of aspects 1 to 9, which is in the form of a food or drink.

The eleventh aspect of the present invention is to provide a composition for cleaning oral cavity, which comprises the composition for antibacterial activity against periodontal disease bacteria as defined in any one of aspects 1 to 9.

According to the tenth and eleventh aspects of the present invention, a food or drink for antibacterial activity against periodontal disease bacteria and a composition for cleaning oral cavity, which prevent periodontal diseases safely and conveniently, can be provided by blending the above-mentioned composition for antibacterial activity against periodontal disease bacteria as the active ingredient.

### Brief Explanation of the Drawings

Fig.1 is a drawing showing time course of the change in the viable cell number when a periodontal disease bacteria *Porphyromonas gingivalis* ATCC 33277 is grown in a culture medium containing a hot water-suspension of whole cocoa fraction.
Fig.2 is a drawing showing time course of the change in the viable cell number when a periodontal disease bacteria *Fusobacterium nucleatum* ATCC 22586 is grown in a culture medium containing a hot water-suspension of whole cocoa fraction.
Fig.3 is a drawing showing time course of the change in the viable cell number when a periodontal disease bacteria *Prevotella intermedia* ATCC 25611 is grown in a culture medium containing a hot water-suspension of whole cocoa fraction.
Fig.4 is a drawing showing time course of the change in the viable cell number when a periodontal disease bacteria *Actinobacillus actinomycetemcomitans* ATCC 33844 is grown in a culture medium containing a hot water-suspension of whole cocoa fraction.
Fig.5 is a drawing showing time course of the change in the viable cell number when a periodontal disease bacteria *Prevotella nigrescens* ATCC 33563 is grown in a culture medium containing a hot water-extract of a cocoa fraction.
Fig.6 is a drawing showing time course of the change in the viable cell number when a periodontal disease bacteria *Fusobacterium nucleatum* ATCC 22586 is grown in a culture medium containing a hot water-extract of a cocoa fraction.
Fig.7 is a drawing showing time course of the change in the viable cell number when a nonpathogenic bacteria indigenous in the oral cavity, *Streptococcus anginosus* ATCC 333977, is grown in a culture medium containing a hot water-suspension of whole cocoa fraction.
Fig.8 is a drawing showing time course of the change in the viable cell number when a nonpathogenic bacteria indigenous in the oral cavity, *Streptococcus salivalius* ATCC 7075, is grown in a culture medium containing a hot water-suspension of whole cocoa fraction.
Fig.9 is a drawing showing time course of the change in the viable cell number when a nonpathogenic bacteria indigenous in the oral cavity, *Streptococcus sanguinis* ATCC 10556, is grown in a culture medium containing a hot water-suspension of whole cocoa fraction.
Fig.10 is a drawing showing time course of the change in the viable cell number when a periodontal disease bacteria *Porphyromonas gingivalis* ATCC 33277 is grown in a culture medium containing a hot water-suspension of whole cocoa fraction.
Fig.11 is a drawing showing time course of the change in the viable cell number when a periodontal disease bacteria *Porphyromonas gingivalis* ATCC 33277 is grown in a culture medium containing either a cocoa solution or a polyphenol fraction (50% methanol extract fraction) extracted from the cocoa solution.
Fig.12 is a drawing showing time course of the change in the viable cell number when a periodontal disease bacteria *Porphyromonas gingivalis* ATCC 33277 is grown in a culture medium containing either PVPP-untreated fraction or PVPP-treated fraction.
Fig.13 is a drawing showing time course of the change in the viable cell number when a periodontal disease bacteria *Porphyromonas gingivalis* ATCC 33277 is grown in a culture medium containing either a polyphenol fraction (50%-methanol extract fraction) extracted from a cocoa solution or a tea-catechin.
Fig.14 is a drawing showing time course of the change in the viable cell number when a periodontal disease bacteria *Porphyromonas gingivalis* ATCC 33277 is grown in a culture medium containing a free fatty acid fraction extracted from a cocoa solution (hexane-methanol extract fraction).
Fig.15 is a drawing showing time course of the change in the viable cell number when a periodontal disease bacteria *Porphyromonas gingivalis* ATCC 33277 is grown in a culture medium containing either a hot water-extract of a cocoa fraction or "FFA comp.".
Fig.16 is a drawing showing time course of the change in the viable cell number when a periodontal disease bacteria *Prevotella nigrescens* ATCC 33563 is grown in a culture medium containing either a hot water-extract of a cocoa fraction or "FFA comp".

### Best Mode of Carrying out the Invention

In the present invention, the cocoa fraction means a component obtained from cacao mass by a removal of cocoa butter, which contains proteins, carbohydrates, dietary fibers (water-soluble hardly digestible polysaccharides, hemicellulose, cellulose, lignin, etc.), phospholipids, inorganic substances (phosphorus, magnesium, calcium, iron, zinc, copper, potassium, sodium, etc.), Vitamin A, Vitamin B family, Vitamin C, Vitamin E (various types of tocopherols, etc.), niacin, oxalic acid, citric acid, malic acid; succinic acid, lactic acid, acetic acid, polyphenols (tannin, epicatechin, catechin, quercetin, etc.), anhydrous caffeine, theobromine, various types of free fatty acids (palmitic acid, stearic acid, oleic acid, linoleic acid, etc.), and the like.

As the cocoa fraction, specifically, cocoa powder obtained from cacao mass by a removal of cocoa butter, or cacao mass itself, may preferably be used.

Further, the hot water-extract of a cocoa fraction in the present invention means a water-soluble fraction that is obtainable from the cocoa fraction by an extraction with hot water. It contains water-soluble components such as the above organic acids, free fatty acids, vitamins and polyphenols, derived from the cocoa fraction. The method for preparing the hot water-extract of a cocoa fraction is not particularly limited. For example, extracts for the hot water-extract of a cocoa fraction may be prepared by taking steps of adding an appropriate amount (usually 5 to 100 times amount of the mass of the cocoa fraction) of water or preferably hot water to cocoa powder or cacao mass, dispersing uniformly, a heat treatment with an autoclave (usually 40 to 130°C for 1 to 30 minutes), and then removing insoluble fractions by filtration, centrifugation or the like. The resulting extracts may be further concentrated, as the case requires, and further dried for use.

Further, the polyphenols derived from a cocoa fraction, for example, may be prepared by taking steps of suspending cocoa or cacao mass in water, adding an equal amount of hexane, thoroughly mixing it, separating an aqueous layer from a hexane layer, recovering the aqueous layer, adding to the recovered aqueous layer an equal amount of 50% (v/v) methanol aqueous solution, thoroughly mixing it, and then recovering the supernatant which methanol is removed.

Further, the free fatty acids extracted from a cocoa fraction in the present invention represent various types of free fatty acids contained in the cocoa fraction, and specifically, palmitic acid, stearic acid, oleic acid, linoleic acid, linolenic acid, palmitoleic acid, myristic acid, lauric acid, etc. may be mentioned. In the present invention, a mixture containing the above various types of free fatty acids may preferably be used. Among them, at least one selected from palmitic acid, stearic acid, oleic acid and linoleic acid may further preferably be used for the mixture. The proportion between the amounts of free fatty acids contained in the mixture is not particularly limited, but it is preferably determined and used on the basis of the proportion between the amounts of the free fatty acids contained in a cocoa fraction. Specifically, a mixture containing 10 to 50 mass% of palmitic acid, 10 to 50 mass% of stearic acid, 10 to 70 mass% of oleic acid, 1 to 50 mass% of linoleic acid, 1 to 10 mass% of linolenic acid, 1 to 10 mass% of palmitoleic acid, 1 to 10 mass% of myristic acid, and 1 to 10 mass% of lauric acid, respectively in the whole amount of free fatty acids, is preferably used.

The mixture containing above respective free fatty acids can be obtained by an extraction of a cocoa fraction with such a method as above-mentioned hot water-extraction, alcohol extraction, hexane extraction, acetone extraction, etc. For example, the above hot water-extract of a cocoa fraction contains respective free fatty acids in a range of the above proportions, and this hot water-extract may be used as it is, and if the case requires, the free fatty acids may be further purified from the hot water-extract. Further, respective free fatty acids may be composed in a predetermined proportion on the basis of the proportion between the amounts of the free fatty acids contained in a cocoa fraction.

The composition for antibacterial activity against periodontal disease bacteria in the present invention includes a cocoa fraction, a hot water-extract of a cocoa fraction, the polyphenols and/or the free fatty acids derived from a cocoa fraction, as active ingredients, for which the above cocoa fraction, hot water-extract of a cocoa fraction, polyphenols and/or free fatty acids derived from a cocoa fraction, may be used as they are. Further, as the case requires, it may contain colorants, preservatives, perfumes, flavors, sweeteners, proteins (lactoprotein, soybean protein, etc.), vitamins, minerals, saccharides (dextrin, etc.), lactic acid bacteria and/or galenicals having antibacterial activity against periodontal disease bacteria, and the like. Among them, it is preferred to contain lactoprotein and/or vitamins, from the nutritional viewpoints.

In case that the composition for antibacterial activity against periodontal disease bacteria in the present invention includes a cocoa fraction as the active ingredient, the cocoa fraction is preferably contained in an amount of 0.01 to 50 mass%, more preferably 0.01 to 10 mass%, in a total amount of the composition.

Further, in case that a hot water-extract of a cocoa fraction is used as the active ingredient, the hot water-extract is preferably contained in an amount of 0.001 to 20 mass%, more preferably 0.1 to 10 mass%, as calculated as solid contents, in a total amount of the composition.

Furthermore, in case that the polyphenols derived from a cocoa fraction are used as the active ingredients, the total of polyphenols is preferably contained in an amount of 0.0001 to 10 mass%, more preferably 0.001 to 1 mass%, in a total amount of the composition.

Further, in case that the free fatty acids derived from a cocoa fraction are used as the active ingredients, the total of free fatty acids is preferably contained in an amount of 0.0001 to 1 mass%, more preferably 0.001 to 0.2 mass%, in a total amount of the composition.

Moreover, in case that the polyphenols and free fatty acids both derived from a cocoa fraction are used in combination as the active ingredients, the total of polyphenols is preferably contained in an amount of 0.0001 to 10 mass%, more preferably 0.01 to 1 mass%; and the total of free fatty acids is contained in an amount of 0.0001 to 1 mass%, more preferably 0.001 to 0.2 mass%, respectively in a total amount of the composition.

Effective dosage for an adult person per day of the composition for antibacterial activity against periodontal disease bacteria in the present invention corresponds to at least 1 g, more preferably 10 g of the cocoa fraction (cacao mass), in terms of active ingredient. Further, it corresponds to 0. 1 to 10 g, more preferably 1 to 10 g of the hot water-extract of a cocoa fraction (solid contents). Furthermore, it corresponds to 0.01 to 1 g, more preferably 0.1 to 1 g of a total of polyphenols derived from a cocoa fraction. Moreover, it corresponds to 0.01 to 1 g, more preferably 0.1 to 1 g of a total of free fatty acids derived from a cocoa fraction.

The composition for antibacterial activity against periodontal disease bacteria in the present invention, as explained in the examples mentioned below, enables to efficiently eradicate the cells of the periodontal disease bacteria belonging to *genus Porphyromonas, genus Prevotella, genus Fusobacterium* or *genus Actinobacillus* those are detected from the focus of periodontal disease at a high rate in patients, without inhibiting the growth of nonpathogenic bacteria indigenous in the oral cavity. Accordingly, it does not adversely influence the balance of the flora of indigenous bacteria in the oral cavity.

As the periodontal disease bacteria belonging to *genus Porphyromonas*, for example, *Porphyromonas gingivalis, Porphyromonas asaccharolytica, Porphyromonas endodontalis*, etc., may be mentioned. Further, as the periodontal disease bacteria belonging to *genus Prevotella*, for example, *Prevotella intermedia, Prevotella nigrescens, Prevotella melaninogenica*, etc., may be mentioned. Furthermore, as the periodontal disease bacteria belonging to genus *Fusobacterium*, for example, *Fusobacterium nucleatum, Fusobacterium necrophorum, Fusobacterium naviforme* AA, etc., may be mentioned. Moreover, as the periodontal disease bacteria belonging to *genus Actinobacillus,* for example, *Actinobacillus actinomycetemcomitans*, etc., may be mentioned.

On the other hand, as the nonpathogenic indigenous bacteria in the oral cavity, *Streptococcus anginosus, Streptococcus salivalius, Streptococcus sanguinis*, etc., may be mentioned for example.

The pharmaceutical form of the composition for antibacterial activity against periodontal disease bacteria in the present invention is not particularly limited. Tablets, pills, powdered drugs, liquid drugs, suspensions, emulsions, granules, etc. may, for example, be mentioned, and these may be chosen depending upon the mode of use.

As the carriers for solid pharmaceutical forms, for example, excipients such as lactose, sucrose, glucose, starch, kaolin, crystalline cellulose and silica, binders such as glucose liquid, starch liquid, gelatin solution, carboxymethylcellulose and hydroxypropylcellulose, disintegrators such as carboxymethylcellulose sodium and low substitution rate hydroxypropylcellulose, surfactants such as polyoxyethylene sorbitan fatty acid ester, lubricants such as stearate, and the like may be used. In the case of tablets, sugar-coated tablets or film-coated tablets may be formed as the case requires.

Further, for preparation in the form of liquid drug, emulsion, suspension, etc., for example, usual solubilizing adjuvants, buffering agents, etc. such as water, ethyl alcohol, macrogol, propylene glycol, polyoxylated isostearyl alcohol and polyoxyethylene sorbitan fatty acid ester, may be used as a solvent. However, propylene glycol is particularly preferably used from the viewpoint of balance with physico-chemical properties of the hot water-extract of a cocoa fraction.

The composition for antibacterial activity against periodontal disease bacteria of the present invention may be added to various foods or drinks such as chocolate, cocoa drink (including jelly drink), gum, candy (hard candy or soft candy), tablet confectionery and various types of retort foods.

The amount of the composition for antibacterial activity against periodontal disease bacteria to be added to a food or drink, may appropriately be set depending upon the above effective dosage, but it preferably corresponds to 0.01 to 50 mass%, more preferably 5 to 30 mass% of a cocoa fraction (cacao mass), in a total amount of the food or drink. Further, it preferably corresponds to 0.1 to 20 mass%, more preferably 0.2 to 10 mass% of a hot water-extract of a cocoa fraction (solid contents), in a total amount of the food or drink. Furthermore, it preferably corresponds to 0.001 to 5 mass%, more preferably 0.1 to 3 mass% of a total of polyphenols derived from a cocoa fraction in a total amount of the food or drink. Moreover, it preferably corresponds to 0.01 to 1 mass%, more preferably 0.05 to 0.5 mass% of a total of free fatty acids derived from a cocoa fraction in a total amount of the food or drink.

Further, the composition for antibacterial activity against periodontal disease bacteria in the present invention, for example, may be added to a composition for cleaning oral cavity, such as dentifrice and mouthwash. The amount of the composition for antibacterial activity against periodontal disease bacteria in the composition for cleaning oral cavity, may appropriately be set depending upon the above effective dosage as well, but it preferably corresponds to 0. 01 to 10 mass%, more preferably 0.1 to 5 mass% of a cocoa fraction, in a total amount of the composition for cleaning oral cavity. Further, it preferably corresponds to 0.001 to 3 mass%, more preferably 0.01 to 2 mass% of a hot water-extract of a cocoa fraction (solid contents), in a total amount of the composition for cleaning oral cavity. Furthermore, it preferably corresponds to 0.0001 to 10 mass%, more preferably 0.001 to 1 mass% of a total of polyphenols derived from cocoa fraction, in a total amount of the composition for cleaning oral cavity. Moreover, it preferably corresponds to 0.001 to 1 mass%, more preferably 0.01 to 0.5 mass% of a total of free fatty acids derived from a cocoa fraction, in a total amount of the composition for cleaning oral cavity.

### EXAMPLES

The present invention will be explained in further detail with reference to examples as follows.

Example 1 (study on effectiveness of cocoa powder for antibacterial activity against periodontal disease bacteria)

Cocoa powder (trade name: "Morinaga Pure Cocoa", produced by Morinaga & Co., Ltd.) was suspended in distilled water so that it would be either 30% (w/v) or 10% (w/v), and treated with an autoclave at 121°C for 15 minutes. The resulting solution was defined as a hot water-suspension of 30% (w/v) whole cocoa fractions or 10% (w/v) whole cocoa fractions, and subjected to the following method for measurement of antibacterial activity.

In order to prepare a culture medium for the assay, a liquid culture medium of BHI (Brain Heart Infusion) medium (produced by Difco Laboratories), supplemented with Yeast Extract (produced by Difco Laboratories) with concentration of 5% (w/v) and by L-cysteine (produced by Sigma Co.) with concentration of 0.05% (w/v), was added with either of the above-mentioned hot water-suspension of whole cocoa fractions blended to 10% (v/v) of total volume. To make a control medium, distilled water was added to the culture medium in place of the hot water-suspension of whole cocoa fractions.

Into respective culture medium, periodontal disease bacteria (*Porphyromonas gingivalis* ATCC 33277, *Fusobacterium nucleatum* ATCC 22486, *Prevotella intermedia* ATCC 25611 or *Actinobacillus actinomycetemcomitans* ATCC 33844) was suspended so that it would be about 10⁵ CFU/ml, and cultured at 37°C under anaerobic condition.

One, 3 or 6 hour(s) after the initiation of the culturing, a fraction of the culture was taken and aliquoted into the step-by-step dilutions, and then a constant amount of each dilution was inoculated onto a hemin menadione-containing Brucella blood agar plate medium in case for *Porphyromonas gingivalis* ATCC 33277 and *Prevotella intermedia* ATCC 25611, and onto a BHI agar plate medium in case for *Fusobacterium nucleatum* ATCC 22486 and *Actinobacillus actinomycetemcomitans* ATCC 33844. The number of the colonies appeared on each plate was counted.

Estimated from the count, the viable cell number in 1 ml of the culture was determined, and the percentage (%CFU) relative to the initial viable cell number was determined.

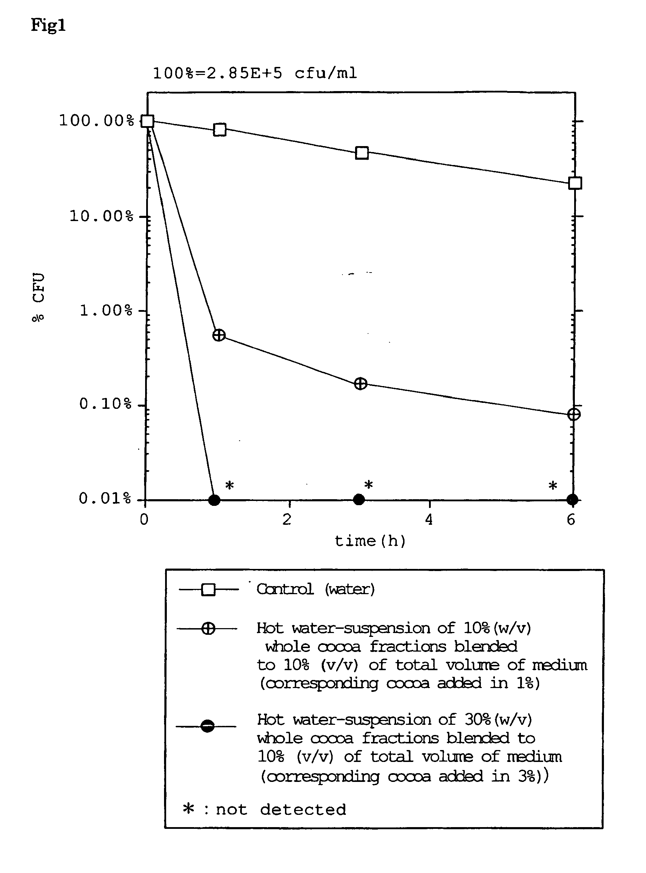

Fig. 1 shows the results of *Porphyromonas gingivalis* ATCC 33277,

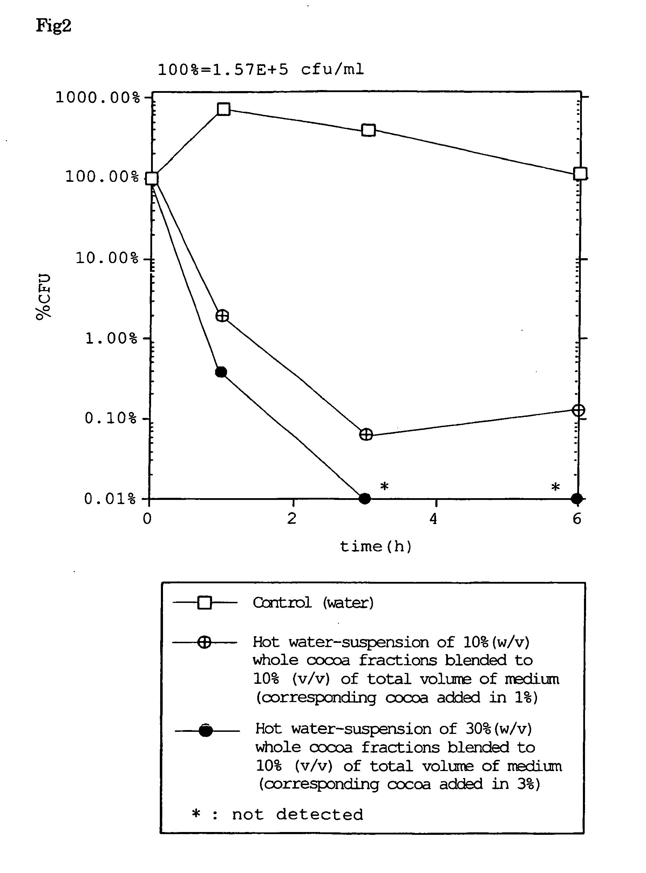

Fig.2 shows the results of *Fusobacterium nucleatum* ATCC 22486,

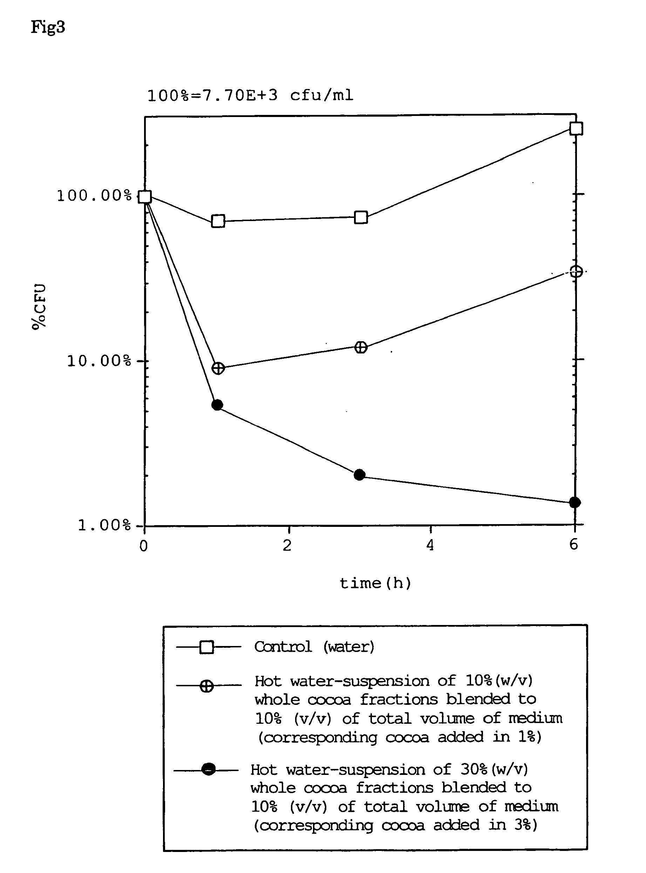

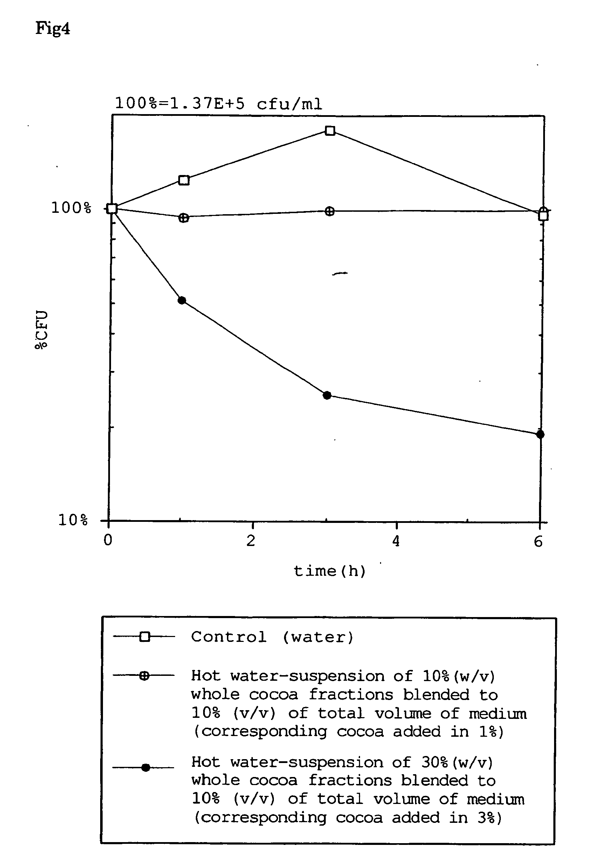

Fig.3 shows the results of *Prevotella intermedia* ATCC 25611, and Fig.4 shows the results of *Actinobacillus actinomycetemcomitans* ATCC 33844.

As shown in Fig.1, *Porphyromonas gingivalis* ATCC 33277 exhibited great decrease of the viable cell number as the period of the culture being extended in either culture medium with the hot water-suspension of 30% (w/v) or 10% (w/v) whole cocoa fractions blended to 10% (v/v) of total volume.

As further shown in Fig.2, *Fusobacterium nucleatum* ATCC 22486 exhibited great decrease of the viable cell number as the period of the culture being extended in either culture medium with the hot water-suspension of 30% (w/v) or 10% (w/v) whole cocoa fractions blended to 10% (v/v) of total volume.

Further, as shown in Fig.3, *Prevotella intermedia* ATCC 25611 exhibited decrease of the viable cell number as the period of the culture being extended in either culture medium with the hot water-suspension of 30% (w/v) or 10% (w/v) whole cocoa fractions blended to 10% (v/v) of total volume, to some extent not so large as either *Porphyromonas gingivalis* ATCC 33277 or *Fusobacterium nucleatum* ATCC 22486 exhibited.

Furthermore, as shown in Fig.4, *Actinobacillus actinomycetemcomitans* ATCC 33844 exhibited decrease of the viable cell number as the period of the culture being extended in either culture medium with the hot water-suspension of 30% (w/v) or 10% (w/v) whole cocoa fractions blended to 10% (v/v) of total volume, as like *Prevotella intermedia* ATCC 25611, to some extent not so large as either *Porphyromonas gingivalis* ATCC 33277 or *Fusobacterium nucleatum* ATCC 22486 exhibited.

From the above results, it was found that the hot water-suspension of whole cocoa fractions has excellent antibacterial activity against *Porphyromonas gingivalis* ATCC 33277, *Fusobacterium nucleatum* ATCC 22486, *Prevotella intermedia* ATCC 25611 and *Actinobacillus actinomycetemcomitans* ATCC 33844, which are all known as the periodontal disease bacteria.

Example 2 (study on effectiveness of a hot water-extract of cocoa powder for antibacterial activity against periodontal disease bacteria)

Cocoa powder (trade name: Morinaga Pure Cocoa, produced by Morinaga & Co. , Ltd.) was suspended in water so that it would be 10% (w/v), and treated with an autoclave at 121°C for 15 minutes. Next, centrifugation was carried out for 15 minutes (x 1,000 g) at room temperature to recover the supernatant. The resulting supernatant was named as a hot water-extract of a cocoa fraction (containing 2 to 3 mass% of solid contents), and subjected to the following method for measurement of antibacterial activity against periodontal disease bacteria.

In order to prepare a culture medium for the assay, to a liquid culture medium of BHI (Brain Heart Infusion) medium (produced by Difco Laboratories), the above-mentioned hot water-extract of a cocoa fraction was added in 1% (v/v) or 10% (v/v) of the total volume. To make a control medium, distilled water was added to the culture medium in place of the hot water-extract of a cocoa fraction.

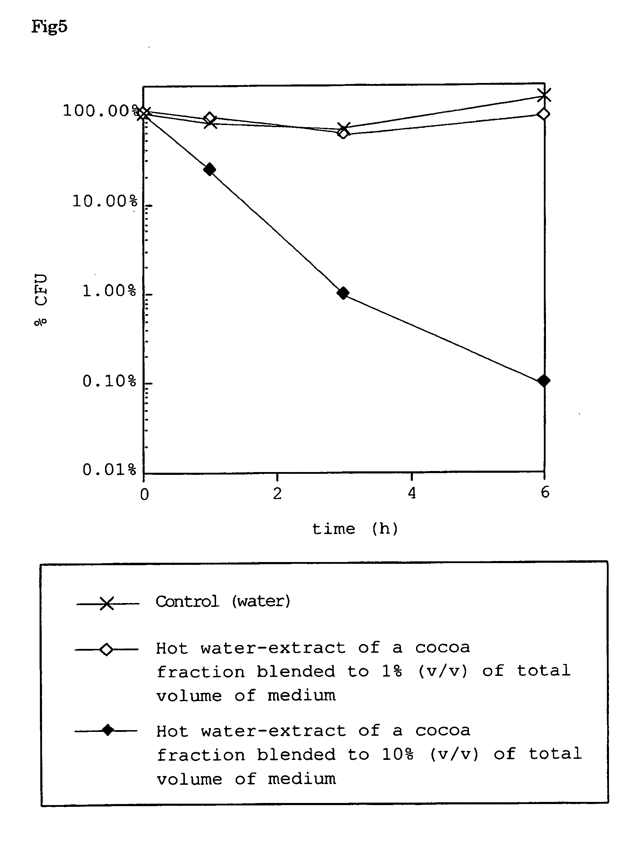

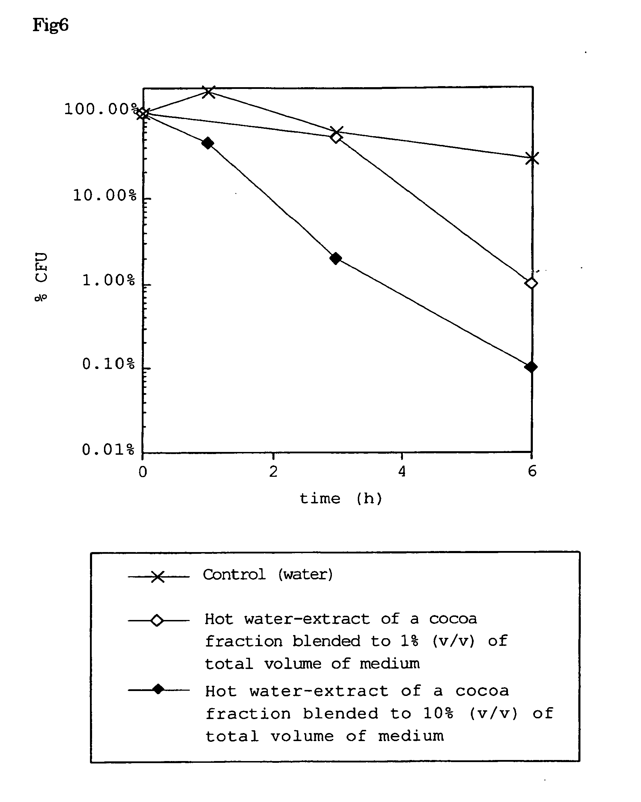

Into respective culture medium, periodontal disease bacteria (*Prevotella nigrescens* ATCC 33563 or *Fusobacterium nucleatum* ATCC 22586) was suspended so that it would be about 10⁶ to 10⁷ CFU/ml, and cultured at 37°C under anaerobic condition. One, 3 or 6 hour(s) after the initiation of the culturing, a fraction of the culture was taken and aliquoted into the step-by-step dilutions, and then a constant amount of each dilution was inoculated onto a hemin menadione-containing Brucella blood agar plate medium. The number of the colonies appeared on each plate was counted. Estimated from the count, the viable cell number in 1 ml of the culture was determined, and the percentage (%CFU) relative to the initial viable cell number was determined. Fig.5 shows the results of *Prevotella nigrescens* ATCC 33563 and Fig.6 shows the results of *Fusobacterium nucleatum* ATCC 22586.

As shown in Fig.5, *Prevotella nigrescens* ATCC 33563 exhibited great decrease of the viable cell number as the period of the culture being extended in a medium containing 10% (v/v) of the hot water-extract of a cocoa fraction.

Further, as shown in Fig. 6, *Fusobacterium nucleatum* ATCC 22586 exhibited great decrease of the viable cell number as the period of the culture being extended in either medium containing 1% (v/v) or 10% (v/v) of hot water-extract of a cocoa fraction,

From the above results, it was found that the hot water-extract of a cocoa fraction has excellent antibacterial activity against *Prevotella nigrescence* ATCC 33563 and *Fusobacterium nucleatum* ATCC 22586 those are both periodontal disease bacteria.

Example 3 (study on influences of cocoa powder onto nonpathogenic bacteria indigenous in the oral cavity)

A BHI-based culture medium was prepared in the same manner as in Example 1, to which the hot water-suspension of 30% (w/v) whole cocoa fractions was blended in 10% (v/v) of total volume. To make a control medium, distilled water was added to the culture medium in place of the hot water-suspension of whole cocoa fractions.

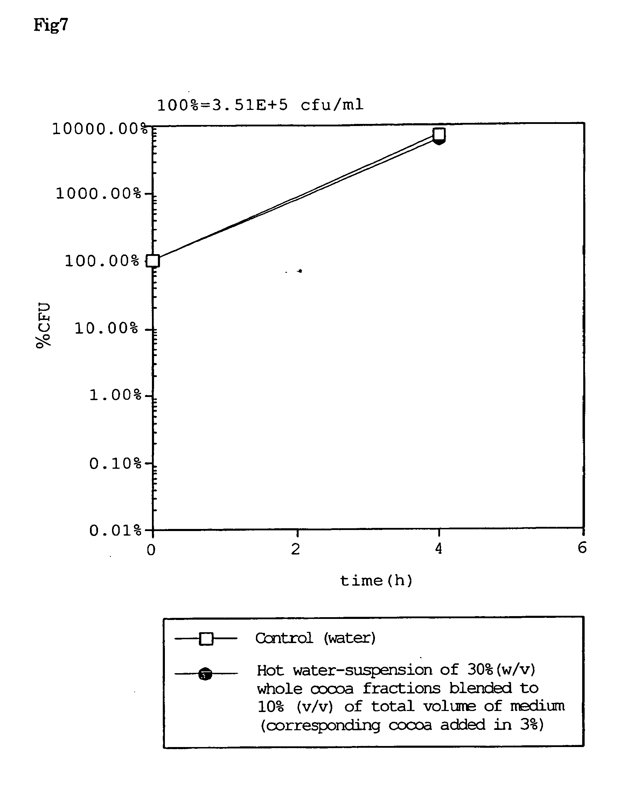

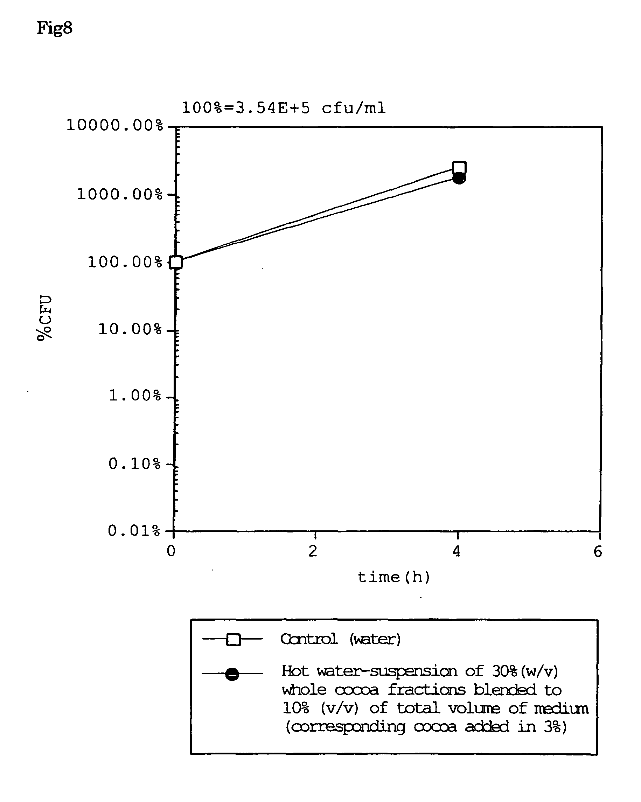

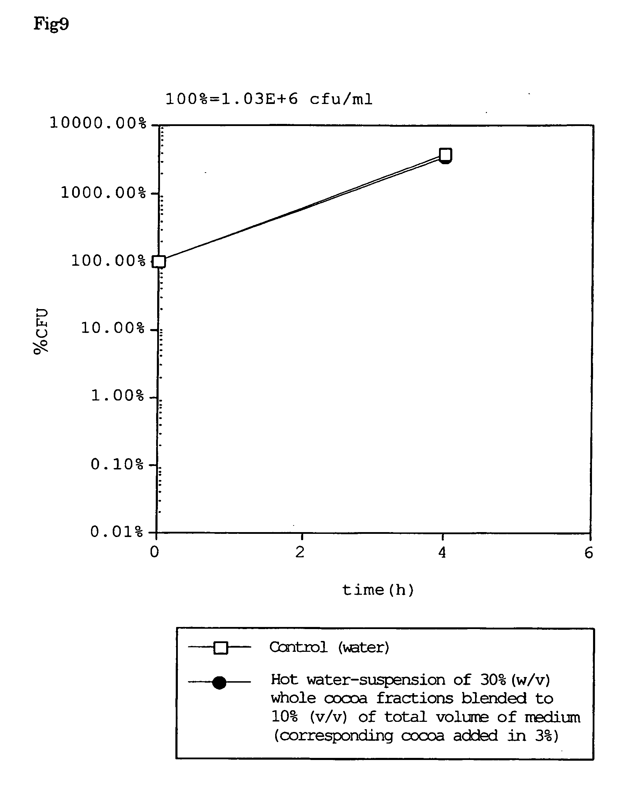

Into the culture medium, nonpathogenic bacteria indigenous in the oral cavity (*Streptococcus anginosus* ATCC 333977, *Streptococcus salivalius* ATCC 7075 or *Streptococcus sanguinis* ATCC 10556) was suspended so that it would be about 10⁵ CFU/ml, and cultured at 37°C under anaerobic condition. Four hours after the initiation of the culturing, a fraction of the culture was taken and aliquoted into the step-by-step dilutions, and then a constant amount of each dilution was inoculated onto a BHI agar plate medium. The number of the colonies appeared on each plate was counted. Estimated from the count, the viable cell number in 1 ml of the culture was determined, and the percentage (%CFU) relative to the initial viable cell number was determined. Fig.7 shows the results of *Streptococcus anginosus* ATCC 33977, Fig.8 shows the results of *Streptococcus salivalius* ATCC 7075, and Fig. 9 shows the results of *Streptococcus sanguinis* ATCC 10556.

As shown in Fig.7, *Streptococcus anginosus* ATCC 33977 exhibited no decrease of the viable cell number as the period of the culture being extended in the culture medium with the hot water-suspension of 30% (w/v) whole cocoa fractions blended to 10% (v/v) of total volume.

As further shown in Fig.8, *Streptococcus salivalius* ATCC 7075 as well exhibited no decrease of the viable cell number as the period of the culture being extended in the culture medium with the hot water-suspension of 30% (w/v) whole cocoa fractions blended to 10% (v/v) of total volume.

As further shown in Fig.9, *Streptococcus sanguinis* ATCC 10556 as well exhibited no decrease of the viable cell number as the period of the culture being extended in the culture medium with the hot water-suspension of 30% (w/v) whole cocoa fractions blended to 10% (v/v) of total volume.

From the above results, it was found that the hot water-suspension of whole cocoa fractions exhibits no antibacterial effects against *Streptococcus anginosus* ATCC 33977, *Streptococcus salivalius* ATCC 7075, and *Streptococcus sanguinis* ATCC 10556, which are all known as the nonpathogenic bacteria indigenous in the oral cavity.

Example 4 (study on effectiveness of cocoa powder for antibacterial activity against periodontal disease bacteria during a short period of time)

A BHI-based culture medium was prepared in the same manner as in Example 1, to which the hot water-suspension of 30% (w/v) whole cocoa fractions was blended in 10% (v/v) of total volume. To make a control medium, distilled water was added to the culture medium in place of the hot water-suspension of whole cocoa fractions.

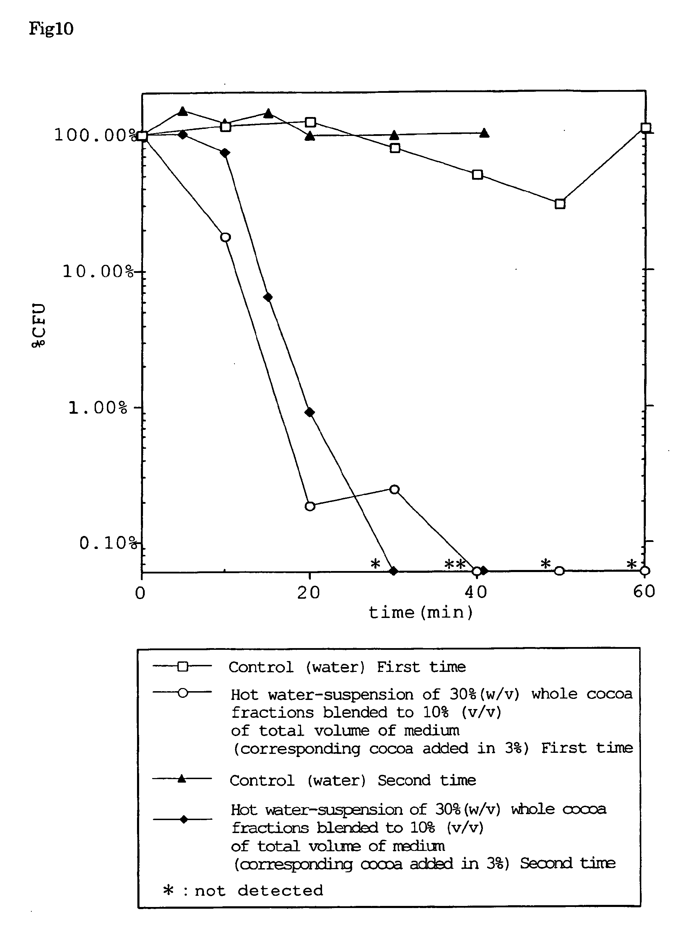

Into the culture medium, *Porphyromonas gingivalis* ATCC 33277 was suspended so that it would be about 10⁵ CFU/ml, and cultured at 37°C under anaerobic condition. Five, 10, 15, 20, 30, 40, 50 or 60 minutes after the initiation of the culturing, a fraction of the culture was taken and aliquoted into the step-by-step dilutions, and then a constant amount of each dilution was inoculated onto a hemin menadione-containing Brucella blood agar plate medium. The number of the colonies appeared on each plate was counted. Estimated from the count, the viable cell number in 1 ml of the culture was determined, and the percentage (%CFU) relative to the initial viable cell number was determined. This experiment was carried out two times. The results are shown in Fig.10.

As shown in Fig.10, *Porphyromonas gingivalis* ATCC 33277 exhibited no substantial decrease of the viable cell number until 10 minutes after the initiation of culturing in the culture medium with the hot water-suspension of 30% (w/v) whole cocoa fractions blended to 10% (v/v) of total volume, but the number decreased to a great extent at 15 minutes after the initiation of culturing, and no viable cell was detected at 30 minutes after the initiation of culturing.

From the above results, it was found that the hot water-suspension of whole cocoa fractions starts exercising its excellent antibacterial activity against *Porphyromonas gingivalis* ATCC 33277 in a short period of time. Accordingly, it is suggested that sufficient antibacterial activity against periodontal disease bacteria can be expected to be effective even by usual drinking of the hot water-suspension of whole cocoa fractions.

Example 5 (study on effectiveness of cocoa powder for antibacterial activity against clinical isolates of periodontal disease bacteria)

A BHI-based culture medium was prepared in the same manner as in Example 1, to which the hot water-suspension of 30% (w/v) or 10% (W/V) whole cocoa fractions was blended in 10% (v/v) of total volume. To make a control medium, distilled water was added to the culture medium in place of the hot water-suspension of whole cocoa fractions.

From the focus of periodontal disease in patients who undergo medical treatment in Tsurumi University Dental Hospital, a mass of periodontal disease bacteria cells was collected by use of a paper point. The bacteria species forming a black-pigmented colony on a hemin menadione-containing Brucella blood agar plate medium was selected, and identified as *Porphyromonas gingivalis* by a PCR method (A. Ashimoto, C. Chen, I. Bakker, J. Slots,. Oral Microbiology Immunology 1996 Aug; 11(4): 266-73). Among them, five of isolates listed in Table 1 (*Porphyromonas gingivalis* 113H, *Porphyromonas gingivalis* 823D, *Porphyromonas gingivalis* 1113C, *Porphyromonas gingivalis* 1113D and *Porphyromonas gingivalis* 1433F) were obtained as clinical isolates of periodontal disease bacteria.

**Table 1**

| Patient's | Male | Female | Female | Female | Female |
|---|---|---|---|---|---|
| sexuality | | | | | |
| Region that | Maxillary | Maxillary | Maxillary | Maxillary | Mandibular |
| isolate | right | right | right | right | right |
| originated | first | first | incisor | incisor | first |
| from | molar | premolar | | | premolar |
| | tooth | tooth | | | tooth |
| Depth of | 6 | 6 | 8 | 8 | 8 |
| periodontal | | | | | |
| pocket (mm) | | | | | |
| Bleeding(BOP) | + | + | + | + | - |
| Pus discharge | - | - | + | + | - |
| Type of | Type IV | Type III | Type II | Type II | Type II |
| flagellum | | | | | |
| Strain name | 113H | 823D | 1113C | 1113D | 1433F |

Into the above-mentioned BHI culture medium, each of the clinical isolates was suspended so that it would be about 10⁵ CFU/ml, and cultured at 37°C under anaerobic condition. One, 3 or 6 hour (s) after the initiation of the culturing, a fraction of the culture was taken and aliquoted into the step-by-step dilutions, and then a constant amount of each dilution was inoculated onto a hemin menadione-containing Brucella blood agar plate medium. The number of the colonies appeared on each plate was counted. Estimated from the count, the viable cell number in 1 ml of the culture was determined, and the percentage (%CFU) relative to the initial viable cell number was determined.

The result was that, all the clinical isolates of *Porphyromonas gingivalis* exhibited great decrease of the viable cell number as the period of the culture being extended in either culture medium with the hot water-suspension of 30% (w/v) or 10% (w/v) whole cocoa fractions blended to 10% (v/v) of total volume, which parallels to the observation with the type strain *Porphyromonas gingivalis* ATCC 33277.

From the above results, it was confirmed that antibacterial activity of cocoa powder against periodontal disease bacteria *Porphyromonas gingivalis* is available for practical use.

Example 6 (Identification of the components having the antibacterial activity in cocoa powder -1)

Cocoa powder (trade name: Red Cocoa, produced by Morinaga & Co., Ltd.) was suspended in distilled water so that it would be 10% (w/v). Hereinafter, this solution is named as a "cocoa solution".

To the "cocoa solution", an equal amount of hexane was added to separate fat and/or contents. To the remaining water-soluble fractions that the fat and/or contents were removed from, an equal amount of 100% methanol was added and shaken for 10 minutes, followed by centrifugation to isolate the supernatant. To the precipitates, 50% methanol was added and shaken for 10 minutes, followed by centrifugation to isolate the supernatant. These operations were repeated two times, and the collected supernatants were applied to an evaporator. After methanol was thoroughly vaporized off, it was suspended in a volume of distilled water so that its concentration would be equivalent to the corresponding concentration in a solution of 10% (w/v) cocoa powder. This fraction is hereinafter named as "50% methanol extract fraction". The polyphenols contained in cocoa are expected to be concentrated in this fraction.

A BHI-based culture medium was prepared, to which either of above-mentioned 10% (w/v) "cocoa solution" or "50% methanol extract fraction" that corresponds to the solution of 10% (w/v) cocoa powder was blended in 10% (v/v) of total volume. To make a control medium, distilled water was added to the culture medium in place of the "cocoa solution" or "50% methanol extract fraction".

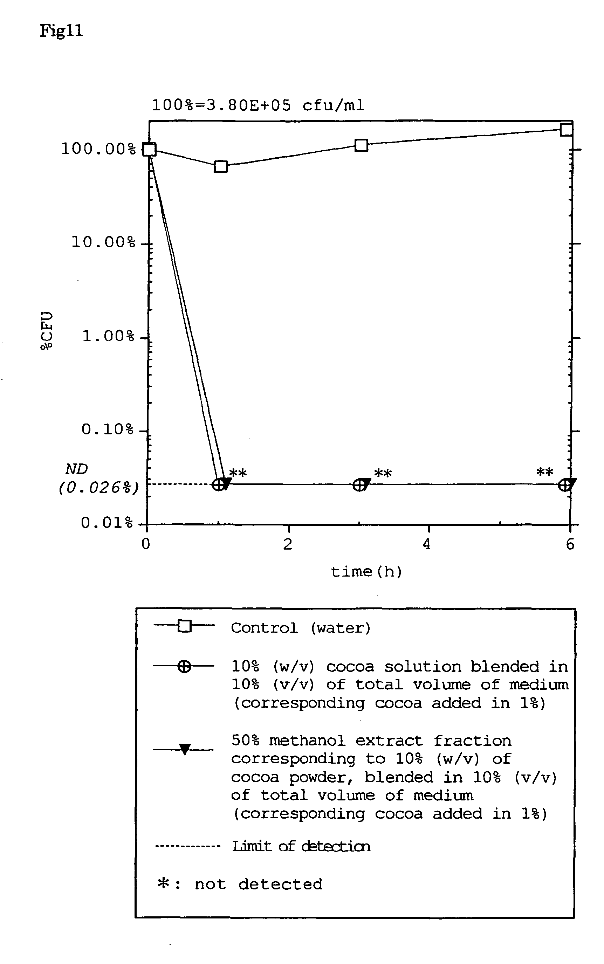

Into the above respective medium, *Porphyromonas gingivalis* ATCC 33277 was suspended so that it would be about 10⁵ CFU/ml, and cultured at 37°C under anaerobic condition. One, 3 or 6 hour(s) after the initiation of the culturing, a fraction of the culture was taken and aliquoted into the step-by-step dilutions, and then a constant amount of each dilution was inoculated onto a hemin menadione-containing Brucella blood agar plate medium. The number of the colonies appeared on each plate was counted. Estimated from the count, the viable cell number in 1 ml of the culture was determined, and the percentage (%CFU) relative to the initial viable cell number was determined. The results are shown in Fig.11.

As shown in Fig.11, *Porphyromonas gingivalis* ATCC 33277 exhibited great decrease of the viable cell number as the period of the culture being extended in either culture medium with 10% (w/v) "cocoa solution" or "50% methanol extract fraction" that corresponds to a solution of 10% (w/v) cocoa powder blended to 10% (v/v) of total volume.

From the above results, polyphenols contained in cocoa were highly likely to be the main component having antibacterial activity. Accordingly, the following experiment was conducted by use of the PVPP that is a resin capable of adsorbing substances having phenol groups, such as polyphenols.

The "50% methanol extract fraction" obtained by the above method was centrifuged, and the resulting water-soluble supernatant was recovered. This fraction is hereinafter named as "PVPP-untreated fraction". To this fraction, PVPP was added in an amount of 1 g/supernatant 10ml, and this was shaken at 37°C for 30 minutes, followed by centrifugation for separation of PVPP, and the supernatant was recovered. This recovered supernatant is hereinafter named as "PVPP-treated fraction".

A BHI-based culture medium was prepared, to which either of the above-mentioned "PVPP-untreated fraction" or "PVPP-treated fraction" was blended in 10% (v/v) of total volume. To make a control medium, distilled water was added to the culture medium in place of the "PVPP-untreated fraction" or "PVPP-treated fraction".

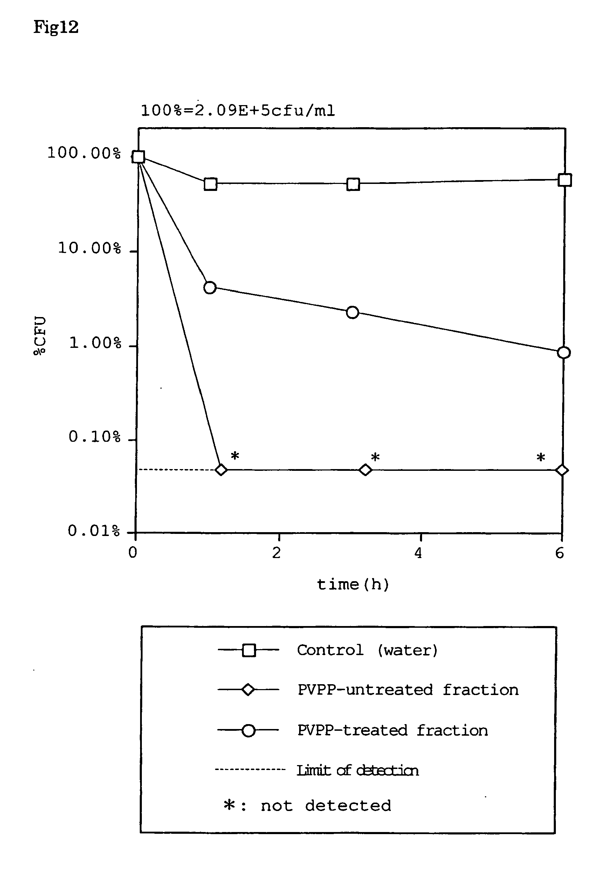

Into the above respective medium, *Porphyromonas gingivalis* ATCC 33277 was suspended so that it would be about 10⁵ CFU/ml, and cultured at 37°C under anaerobic condition. One, 3 or 6 hour (s) after the initiation of the culturing, a fraction of the culture was taken and aliquoted into the step-by-step dilutions, and then a constant amount of each dilution was inoculated onto a hemin menadione-containing Brucella blood agar plate medium. The number of the colonies appeared on each plate was counted. Estimated from the count, the viable cell number in 1 ml of the culture was determined, and the percentage (%CFU) relative to the initial viable cell number was determined. The results are shown in Fig.12.

As shown in Fig.12, *Porphyromonas gingivalis* ATCC 33277 exhibited great decrease of the viable cell number in the culture medium with the "PVPP-untreated fraction" blended to 10% (v/v) of total volume. On the other hand, *Porphyromonas gingivalis* ATCC 33277 did not exhibit great decrease of the viable cell number in the culture medium with the "PVPP-treated fraction" blended to 10% (v/v) of total volume. The result paralleled with the concentration of polyphenols determined from each respective fraction. From the above results, it was confirmed that the polyphenols, which can be adsorbed to the PVPP resin, were main component of cocoa for antibacterial activity.

Example 7 (comparison of the effectiveness in antibacterial activity of polyphenols extracted from cocoa with that of tea-catechin)

The composition (trade name: "Sunphenon", produced by TAIYO KAGAKU CO., LTD.) containing polyphenols including epigallocatechin and the like, which is derived from tea, was suspended in distilled water so that it would be 0.05% (w/v), and then treated by an autoclave at 121°C for 15 minutes. This solution is hereinafter named as "tea-catechin solution".

The concentration of polyphenols contained in the tea-catechin solution was measured and determined as well as that of the "50% methanol extract fraction" prepared in Example 6. The "50% methanol extract fraction" was adjusted to a solution which concentration would be the corresponding concentration in a solution of 1.25% (w/v) cocoa powder, so that it contains polyphenols with equivalent concentration to that of the "tea-catechin solution" (0.313 mg/ml).

A BHI-based culture medium was prepared, to which either of solutions, the "tea-catechin solution" or the adjusted "50% methanol extract fraction" that corresponds to a solution of 1.25% (w/v) cocoa powder, was blended in 10% (v/v) of total volume. To make a control medium, distilled water was added to the culture medium in place of the "tea-catechin solution" or the adjusted "50% methanol extract fraction".

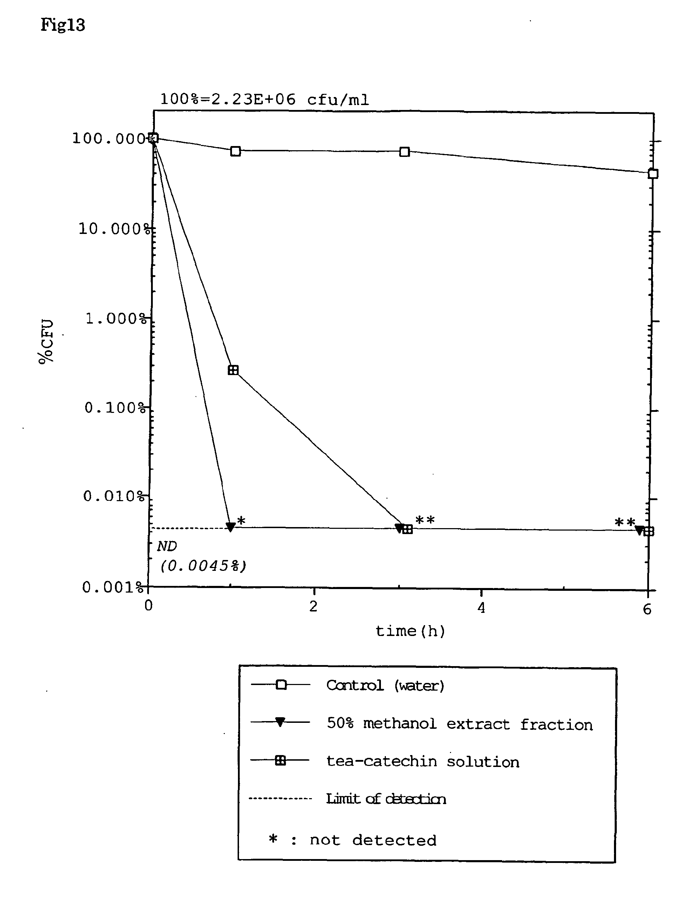

Into the above respective medium, *Porphyromonas gingivalis* ATCC 33277 was suspended so that it would be about 10⁶ CFU/ml, and cultured at 37°C under anaerobic condition. One, 3 or 6 hour (s) after the initiation of the culturing, a fraction of the culture was taken and aliquoted into the step-by-step dilutions, and then a constant amount of each dilution was inoculated onto a hemin menadione-containing Brucella blood agar plate medium. The number of the colonies appeared on each plate was counted. Estimated from the count, the viable cell number in 1 ml of the culture was determined, and the percentage (%CFU) relative to the initial viable cell number was determined. The results are shown in Fig.13.

As shown in Fig.13, no viable cell of *Porphyromonas gingivalis* ATCC 33277 was detected 1 hour after the initiation of culturing in the culture medium with the "50% methanol extraction fraction" that corresponds to 1.25% (w/v) cocoa powder blended to 10% (v/v) of total volume. On the other hand, *Porphyromonas gingivalis* ATCC 33277 exhibited decrease of the viable cell number 1 hour after the initiation of culturing, but kept around 0.3% CFU in the culture medium with the tea-catechin solution blended to 10% (v/v) of total volume. As the period of the culture being further extended, the viable cell number in both culture media decreased to a great extent.

From these results, it is suggested that the polyphenol composition extracted from cocoa has a higher specific activity than that derived from tea, in comparison of the effectiveness in antibacterial activity.

Example 8 (Identification of the components having the antibacterial activity in cocoa powder -2)

A 10% (w/v) "cocoa solution" was adjusted to pH 2 with 2N hydrochloric acid. By this process, free fatty acids those are dissolved in their salt-forms in the water content of the "cocoa solution" are all transferred to fat and/or oil contents.

To this acidified "cocoa solution", an equal amount of hexane was added, and it was shaken at room temperature for 10 minutes, and then followed by centrifugation to separate the hexane fraction. Three times of the stirring and centrifugation process were performed to the solution. The collected hexane fractions were applied to an evaporator, and after hexane was thoroughly vaporized off, it was suspended in a volume of distilled water so that its concentration would be equivalent to the corresponding concentration in a solution of 10% (w/v) cocoa powder. Further, the pH was neutralized to the initial state with 8N sodium hydroxide. To this fraction, 100% methanol of four times volume was added, and this fraction was shaken for 15 minutes at 65°C, and then centrifuged to separate an aqueous layer from an oil layer. Two times of the stirring and centrifugation process were performed to the solution. The collected aqueous layer was applied to an evaporator, and after methanol was thoroughly vaporized off, it was suspended in a volume of distilled water so that its concentration would be equivalent to the corresponding concentration in a solution of 10% (w/v) cocoa powder. This fraction is hereinafter named as "hexane-methanol extract fraction". The free fatty acids contained in cocoa are expected to be concentrated in this fraction.

A BHI-based culture medium was prepared, to which the "hexane-methanol extract fraction" suspension that corresponds to the solution of 10% (w/v) cocoa powder was blended in 10% (v/v) of total volume. To make a control medium, distilled water was added to the culture medium in place of the "hexane-methanol extract fraction" suspension.

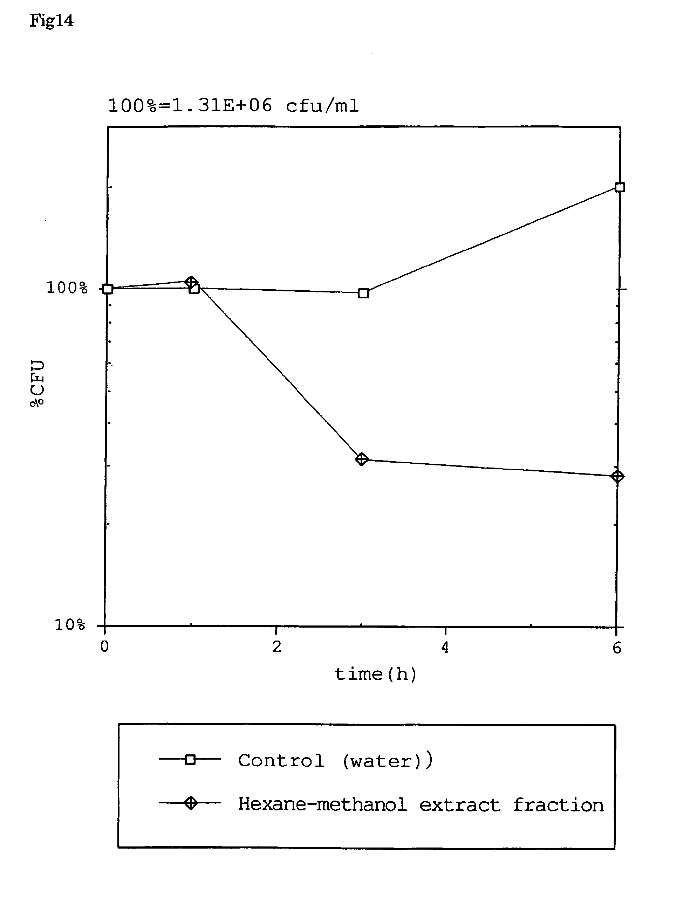

Into the above respective culture medium, *Porphyromonas gingivalis* ATCC 33277 was suspended so that it would be about 10⁵ CFU/ml, and cultured at 37°C under anaerobic condition. One, 3 or 6 hour (s) after the initiation of the culturing, a fraction of the culture was taken and aliquoted into the step-by-step dilutions, and then a constant amount of each dilution was inoculated onto a hemin menadione-containing Brucella blood agar plate medium. The number of the colonies appeared on each plate was counted. Estimated from the count, the viable cell number in 1 ml of the culture was determined, and the percentage (%CFU) relative to the initial viable cell number was determined. The results are shown in Fig.14.

As shown in Fig.14, *Porphyromonas gingivalis* ATCC 33277 exhibited decrease of the viable cell number as the period of the culture being extended in the culture medium with the "hexane-methanol extract fraction" that corresponds to 10% (w/v) of cocoa powder blended to 10% (v/v) of total volume. From the above results, it was supposed that the free fatty acid composition extracted from cocoa also has antibacterial activity.

### Example 9

A BHI-based culture medium was prepared in the same manner as in Example 2, to which the hot water-extract of a cocoa fraction was blended in 10% (v/v) of total volume. To make a control medium, distilled water was added to the culture medium in place of the hot water-extract of a cocoa fraction.

Further, a composition of free fatty acids was prepared by adding four kinds of free fatty acids to purified water in the proportion and concentrations as indicated in Table 2. (hereinafter referred to as "FFA comp."), and this composition was blended in 10% (v/v) of total volume to prepare the other BHI-based culture medium. This "FFA comp." was reconstituted on the basis of proportion and concentrations of free fatty acids contained in a pure cocoa which are determined by mass spectrum analysis and the like, and so the composition of those free fatty acids in "FFA comp." is equivalent to the composition of main four free fatty acids in the hot water-extract of a cocoa fraction.

**Table 2**

| Free fatty acids composition in FFA comp. | µg/ml (µM) |
|---|---|
| Sodium palmitate | 143 (513) |
| Sodium stearate | 128 (418) |
| Sodium oleate | 156 (512) |
| Sodium linolate | 43 (142) |

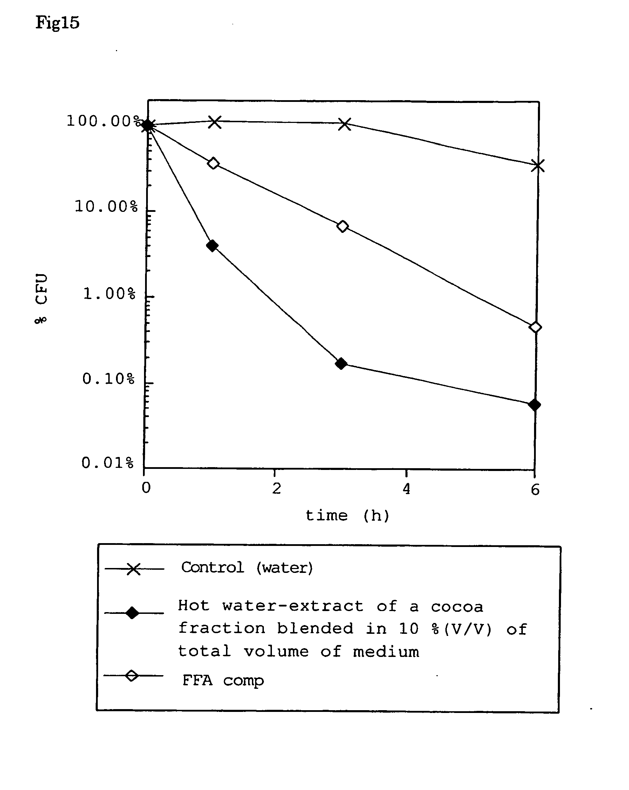

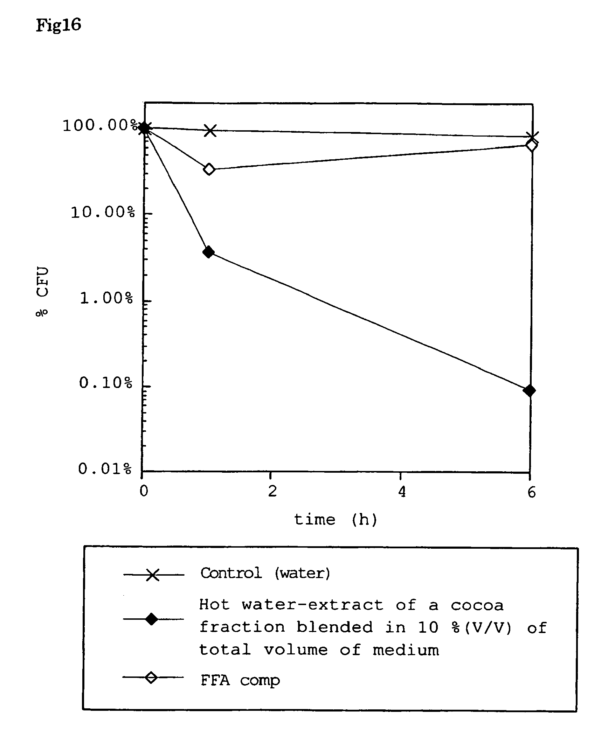

Into these mediums, periodontal disease bacteria (*Porphyromonas gingivalis* ATCC 33277 or *Prevotella nigrescens* ATCC 33563) was suspended so that it would be about 10⁶ to 10⁷ CFU/ml, and cultured at 37°C under anaerobic condition. One, 3 or 6 hour(s) after the initiation of the culturing, a fraction of the culture was taken and aliquoted into the step-by-step dilutions, and then a constant amount of each dilution was inoculated onto a hemin menadione-containing Brucella blood agar plate medium. The number of the colonies appeared on each plate was counted. Estimated from the count, the viable cell number in 1 ml of the culture was determined, and the percentage (%CFU) relative to the initial viable cell number was determined. Fig. 15 shows the results of *Porphyromonas gingivalis* ATCC 33277. Fig.16 shows the results of *Prevotella nigrescens* ATCC 33563.

As shown in Fig.15, *Porphyromonas gingivalis* ATCC 33277 exhibited great decrease of the viable cell number as the period of the culture being extended in the culture medium with a hot water-extract of a cocoa fraction blended to 10% (v/v) of total volume. In a case "FFA comp. " was added to the culture medium, the viable cell number decreased similarly to a great extent with the lapse of culture time.

Further, as shown in Fig.16, *Prevotella nigrescens* ATCC 33563 exhibited great decrease of the viable cell number as the period of the culture being extended in the culture medium with a hot water-extract of a cocoa fraction blended to 10% (v/v) of total volume, which is equivalent to the results shown in Fig.5. In a case "FFA comp." was added to the culture medium, the decrease in the viable cell number was observed as well in comparison with the control medium.

From the above results, it was found that both the hot water-extract of a cocoa fraction and the free fatty acids extracted from cocoa fractions have excellent antibacterial activity against *Porphyromonas gingivalis* ATCC 33277, *Prevotella nigrescens* ATCC 33563 and *Fusobacterium nucleatum* ATCC 22586, although the effective concentrations are varying depending upon the type of periodontal disease bacteria.

### Example 10

Chocolate containing the composition for antibacterial activity against periodontal disease bacteria in the present invention was produced with the formula as indicated in Table 3 below.

**Table 3**

| Materials | Proportion (parts by mass) |
|---|---|
| Sugar | 45.56 |
| Cacao mass | 20 |
| Whole milk powder | 16.5 |
| Cocoa butter | 16.5 |
| Lecithin | 0.4 |
| Vanilla essence | 0.04 |
| Hot water-extract of cocoa | 1 |
| fraction | |
| Total | 100 |

### Example 11

Chewing gum containing the composition for antibacterial activity against periodontal disease bacteria in the present invention was produced with the formula as indicated in Table 4 below.

**Table 4**

| Materials | Proportion (parts by mass) |
|---|---|
| Gum base | 20 |
| Sugar | 53.75 |
| Glucose | 15 |
| Sugar syrup | 7 |
| Hot water-extract of cocoa | 4.25 |
| fraction | |
| Total | 100 |

### Example 12

Candy containing the composition for antibacterial activity against periodontal disease bacteria in the present invention was produced with the formula as indicated in Table 5 below.

**Table 5**

| Materials | Proportion (parts by mass) |
|---|---|
| Sugar | 50 |
| Sugar syrup | 33 |
| Citric acid | 1 |
| Hot water-extract of cocoa | 2.5 |
| fraction | |
| Water | 13.5 |
| Total | 100 |

### Example 13

A sports drink (isotonic drink) containing the composition for antibacterial activity against periodontal disease bacteria in the present invention was produced with the formula as indicated in Table 6 below.

**Table 6**

| Materials | Proportion (parts by mass) |
|---|---|
| Orange syrup | 0.2 |
| Sugar | 1.8 |
| Isomerized glucose syrup | 5.5 |
| (F-55) | |
| Citric acid | 0.14 |
| Common salt | 0.08 |
| Sodium citrate | 0.07 |
| Potassium chloride | 0.04 |
| Calcium primary phosphate | 0.013 |
| Sodium glutamate | 0.004 |
| Magnesium chloride | 0.003 |
| Ascorbic acid | 0.1 |
| Cloudy | 0.1 |
| Emulsified flavor | 0.01 |
| Essence | 0.2 |
| Hot water-extract of cocoa | 1.00 |
| fraction | |
| Water | Residue |
| Total | 100 |

### Example 14

Royal jelly drink containing the composition for antibacterial activity against periodontal disease bacteria in the present invention was produced with the formula as indicated in Table 7 below.

**Table 7**

| Materials | Proportion (parts by mass) |
|---|---|
| Isomerized glucose syrup | 12 |
| (F-55) | |
| Purified honey | 11 |
| Crude royal jelly | 4.5 |
| Garlic extract | 0.2 |
| Bracket fungus extract | 0.3 |
| Citric acid | 0.1 |
| Polydextrose | 4 |
| Natural caffeine | 0.08 |
| Ascorbic acid | 0.5 |
| Vitamin B1 hydrochloride | 0.02 |
| Vitamin B2 phosphate | 0.01 |
| Vitamin B6 hydrochloride | 0.03 |
| Nicotinamide | 0.04 |
| Cloudy | 0.1 |
| Essence | 0.4 |
| Hot water-extract of cocoa | 0.1 |
| fraction | |
| Water | Residue |
| Total | 100 |

### Example 15

Azuki bean gruel packed in a retort pouch containing the composition for antibacterial activity against periodontal disease bacteria of the present invention was produced with the formula as indicated in Table 8 below.

**Table 8**

| Materials | Proportion (parts by mass) |
|---|---|
| Unpolished rice | 4.2 |
| Polished rice | 4.8 |
| Azuki bean | 1.6 |
| Sugar | 0.5 |
| Common salt | 0.1 |
| Hot water-extract of cocoa | 2 |
| fraction | |
| Water | Residue |
| Total | 100 |

### Example 16

Troche containing the composition for antibacterial activity against periodontal disease bacteria in the present invention was produced with the formula as indicated in Table 9 below.

**Table 9**

| Materials | Proportion (parts by mass) |
|---|---|
| Maltitol | 74.3 |
| Lactose | 17.5 |
| Gum arabic | 6.0 |
| Sodium monofluorophosphate | 0.7 |
| Menthol | 0.5 |
| Hot water-extract of cocoa | 1 |
| fraction | |
| Total | 100 |

### Example 17

Mouthwash containing the composition for antibacterial activity against periodontal disease bacteria in the present invention was produced with the formula as indicated in Table 10 below.

**Table 10**

| Materials | Proportion (parts by mass) |
|---|---|
| Ethanol | 20 |
| Saccharin | 0.05 |
| Sodium copper chlorophyllin | 0.1 |
| Water | 78.6 |
| Hot water-extract of cocoa | 1.25 |
| fraction | |
| Total | 100 |

### Example 18

Toothpaste containing the composition for antibacterial activity against periodontal disease bacteria in the present invention was produced with the formula as indicated in Table 11 below.

**Table 11**

| Materials | Proportion (parts by mass) |
|---|---|
| Calcium carbonate | 50 |
| Glycerol | 20 |
| Carrageenan | 0.5 |
| Carboxymethyl cellulose | 1 |
| Lauryl ethanol amide | 1 |
| Sucrose monolaurate | 2 |
| Chlorhexidine hydrochloride | 0.01 |
| Saccharin | 0.05 |
| Sodium copper chlorophyllin | 0.1 |
| Water | 24.3 |
| Hot water-extract of cocoa | 1 |
| fraction | |
| Total | 100 |

### Industrial applicability

According to the present invention as explained above, the composition for antibacterial activity against periodontal disease bacteria, which has no side effect and is safe, exhibiting excellent antibacterial effects, can be provided by being blended as active ingredients with a cocoa fraction contained in cacao mass, extracts from the cocoa fraction by extraction with hot water, polyphenols derived from the cocoa fraction, and/or free fatty acids derived from the cocoa fraction. This composition for antibacterial activity against periodontal disease bacteria has quick-acting antibacterial activity against periodontal disease bacteria belonging to *genus Porphyromonas, genus Prevotella, genus Fusobacterium* or *genus Actinobacillus* those are detected from the focus of periodontal disease at high rate in patients. The composition is expected to exhibit preventive or therapneutic effects against periodontal disease such as periodontitis or periodontoclasia by inhibition of the growth of the bacteria. Further, it does not inhibit the growth of nonpathogenic bacteria indigenous in the oral cavity, and is useful for improvement of the balance of indigenous bacteria flora in the oral cavity

According to the present invention, a food or drink, a composition for cleaning oral cavity, each of which enables to prevent periodontal disease safely and conveniently, can be also provided by being blended with the above-mentioned composition for antibacterial activity against periodontal disease bacteria.

## Claims

1. A composition comprising a cocoa fraction as an active ingredient derived from cacao mass for use in treating or preventing periodontal disease caused by periodontal disease bacteria belonging to *genus Porphyromonas, genus Prevotella, genus Fusobacterium* or *genus Actinobacillus.*

2. A composition for use according to claim 1, wherein the active ingredient comprises a hot water-extract of a cocoa fraction.

3. A composition for use according to claim 1 or claim 2, wherein the active ingredient comprises polyphenols extracted from a cocoa fraction.

4. A composition for use according to any of claims 1 to 3, wherein the active ingredient comprises free fatty acids extracted from a cocoa fraction.

5. The composition for use according to claim 4, wherein the free fatty acid is at least one selected from palmitic acid, stearic acid, oleic acid and linoleic acid.

6. The composition for use according to any one of claims 1 to 5, wherein the cocoa fraction is cocoa powder and/or cacao mass.

7. A composition for use according to any one of claims 1 to 6, which is in the form of a food or drink.

8. A composition for use according to any one of claims 1 to 6, which is in the form of a tablet, a pill, a powder, a liquid, a suspension, an emulsion, or a granule.

9. A composition for use according to any one of claims 1 to 6, which is in the form of a dentifrice or mouthwash.

## Patentansprüche

1. Zusammensetzung, umfassend eine Kakaofraktion als Wirkstoff, erhalten aus Kakaomasse, zur Verwendung bei der Behandlung oder Vorbeugung von Parodontalerkrankung, die durch Parodontalerkrankung-Bakterien, die zu *genus Porphyromonas, genus Prevotella, genus Fusobacterium* oder *genus Actinobacillus* gehören, verursacht wird.

2. Zusammensetzung zur Verwendung gemäß Anspruch 1, wobei der Wirkstoff einen Heißwasserextrakt einer Kakaofraktion umfasst.

3. Zusammensetzung zur Verwendung gemäß Anspruch 1 oder Anspruch 2, wobei der Wirkstoff Polyphenole, die aus einer Kakaofraktion extrahiert sind, umfasst.

4. Zusammensetzung zur Verwendung gemäß einem der Ansprüche 1 bis 3, wobei der Wirkstoff freie Fettsäuren, die aus einer Kakaofraktion extrahiert sind, umfasst.

5. Zusammensetzung zur Verwendung gemäß Anspruch 4, wobei die freie Fettsäure wenigstens eine, ausgewählt aus Palmitinsäure, Stearinsäure, Oleinsäure und Linolensäure, ist.

6. Zusammensetzung zur Verwendung gemäß einem der Ansprüche 1 bis 5, wobei die Kakaofraktion Kakaopulver und/oder Kakaomasse ist.

7. Zusammensetzung zur Verwendung gemäß einem der Ansprüche 1 bis 6, die in der Form eines Nahrungsmittels oder Getränks vorliegt.

8. Zusammensetzung zur Verwendung gemäß einem der Ansprüche 1 bis 6, die in der Form einer Tablette, einer Pille, eines Pulvers, einer Flüssigkeit, einer Suspension, einer Emulsion oder eines Granulats vorliegt.

9. Zusammensetzung zur Verwendung gemäß einem der Ansprüche 1 bis 6, die in der Form einer Zahnpaste oder eines Mundwassers vorliegt.

## Revendications

1. Composition comprenant une fraction de cacao en tant qu'ingrédient actif, dérivant d'une pâte de cacao, pour une utilisation dans le traitement ou la prévention d'une maladie parodontale provoquée par des bactéries de maladies parodontales appartenant au genre *Porphyromonas*, au genre *Prevotella*, au genre *Fusobacterium* ou au genre *Actinobacillus*.

2. Composition pour utilisation selon la revendication 1, dans laquelle l'ingrédient actif comprend un extrait à l'eau chaude d'une fraction de cacao.

3. Composition pour utilisation selon la revendication 1 ou la revendication 2, dans laquelle l'ingrédient actif comprend des polyphénols extraits d'une fraction de cacao.

4. Composition pour utilisation selon l'une quelconque des revendications 1 à 3, dans laquelle l'ingrédient actif comprend des acides gras libres extraits d'une fraction de cacao.

5. Composition pour utilisation selon la revendication 4, dans laquelle l'acide gras libre est au moins l'un choisi parmi l'acide palmitique, l'acide stéarique, l'acide oléique et l'acide linoléique.

6. Composition pour utilisation selon l'une quelconque des revendications 1 à 5, dans laquelle la fraction de cacao est de la poudre de cacao et/ou de la pâte de cacao.

7. Composition pour utilisation selon l'une quelconque des revendications 1 à 6, qui est sous la forme d'un aliment ou d'une boisson.

8. Composition pour utilisation selon l'une quelconque des revendications 1 à 6, qui est sous la forme d'un comprimé, d'une pilule, d'une poudre, d'un liquide, d'une suspension, d'une émulsion, ou d'un granule.

9. Composition pour utilisation selon l'une quelconque des revendications 1 à 6, qui est sous la forme d'un dentifrice ou d'un bain de bouche.
